# EUROPEAN PATENT SPECIFICATION

(11) **EP 1 743 177 B1**
(45) Date of publication and mention of the grant of the patent: **26.03.2008**
(21) Application number: 05746225.1
(22) Date of filing: 05.05.2005
(51) Int. Cl.: G01N 33/68

(54) **METHOD FOR CHARACTERISING COMPOUNDS**
VERFAHREN ZUR CHARAKTERISIERUNG VON VERBINDUNGEN
PROCEDE DE CARACTERISATION DE COMPOSES

(30) Priority: 06.05.2004 GB 0410105
(43) Date of publication of application: 17.01.2007
(73) Proprietor: GE Healthcare UK Limited, Little Chalfont Buckinghamshire HP7 9NA (GB)
(72) Inventor: THOMAS, Nicholas, Whitchurch, Cardiff CF14 7YT (GB); STUBBS, Simon, Whitchurch, Cardiff CF14 7YT (GB); ISMAIL, Rahman, Whitchurch, Cardiff CF14 7YT (GB); MICHAEL, Paul, Whitchurch, Cardiff CF14 7YT (GB); KENRICK, Michael, Whitchurch, Cardiff CF14 7YT (GB); KENDALL, Jonathan, Whitchurch, Cardiff CF14 7YT (GB)
(74) Representative: Hesslewood, Ian Philip
(86) International application number: PCT/GB2005/001705
(87) International publication number: WO 2005/109003

(56) References cited:
- EP-A- 1 022 335
- US-A1- 2003 059 794
- HARTIG P C ET AL: "Development of two androgen receptor assays using adenoviral transduction of MMTV-luc reporter and/or hAR for endocrine screening" TOXICOLOGICAL SCIENCES, vol. 66, no. 1, March 2002 (2002-03), pages 82-90, XP002361139 ISSN: 1096-6080
- LOHSE MARTIN J ET AL: "Direct optical recording of intrinsic efficacy at a G protein-coupled receptor." LIFE SCIENCES, vol. 74, no. 2-3, 5 December 2003 (2003-12-05), pages 397-404, XP002361140 ISSN: 0024-3205
- MOORE KEITH ET AL: "Cell-based versus isolated target screening: How lucky do you feel?" JOURNAL OF BIOMOLECULAR SCREENING, vol. 6, no. 2, April 2001 (2001-04), pages 69-74, XP002361141 ISSN: 1087-0571

## Description

### Technical Field

The present invention relates to methods for characterising or profiling the effects of compounds or molecules on signalling pathways and cellular processes.

### Background to the Invention

The development of new drugs commonly entails evaluation of large numbers of chemical compounds in high-throughput primary screening followed by more detailed examination of compound activity and specificity in secondary screening. Primary screening analyses are typically simple molecular assays such as ligand binding and provide only low level information on the activity of test molecules for selection of the most active compounds for further evaluation. Consequently it is during secondary screening that decisions are taken on compound progression based on a variety of functional assays which may address mode of action, specificity, cellular toxicity and other parameters. Since compound progression becomes increasingly expensive beyond this point, effective characterisation or profiling of lead compounds and selection of compounds with optimal properties is a key activity within the overall drug discovery process. To achieve effective lead profiling there is a requirement for secondary screening assays to provide a comprehensive and detailed survey of compound characteristics; hence there is a need in the drug discovery industry for a means to efficiently apply a diverse panel of lead profiling cellular assays to a number of lead compounds in parallel.

A variety of approaches to compound profiling using arrays of molecular assays have been devised. US 5811231 describes a method for identifying toxic disturbances to the biochemical and biophysical homeostasis of a cell caused by a compound. US 6509153 provides methods for the determination of the potential toxicity of test compounds using nucleic acid hybridisation to determine expression of genes characteristic of deregulated cell viability or proliferation in cells. WO03042654 provides a method of selecting one or more genes that are indicative of an effective therapy by deriving a score value for each of the disease signature and drug signature genes identified to provide an indication of a successful drug for treating the disease. WO0039336 provides methods of characterizing drug activities using consensus profiles whereby response profiles for a biological response of interest are obtained which are associated with drug effectiveness or toxicity.

The preceding methods are jointly characterised in profiling compounds by analysing gene expression patterns. Such patterns may be indicative of lead compound activity but do not provide direct information on the target or nature of compound activity against defined cellular pathways and processes. Alternative approaches to lead profiling have taken a more direct approach to characterising compound activity by using analyses based on specific read-outs in cellular assays.

WO0234881 provides methods for generating and using stable cell lines with integrated reporter genes for screening libraries of drug candidates to identify potential therapeutic agents and for identifying genes which regulate the integrated reporter. US5928888 provides methods for identifying proteins or compounds that directly or indirectly modulate gene expression by insertion of a beta-lactamase reporter gene into the genome of a cell, contacting the cell with a predetermined concentration of a modulator, and detecting beta-lactamase activity in the cell. WO9706277 describes methods for estimating the physiological specificity of a candidate drug by detecting reporter gene product signals from each of a plurality of different, separately isolated cells of a target organism, where each cell contains a reporter gene linked to a different endogenous transcriptional regulatory element, and the sum of the cells comprises an ensemble of the transcriptional regulatory elements of the organism sufficient to estimate of the physiological specificity of the candidate drug.

While these assays provide a more direct indication of compound activity than those approaches using broad surveys of gene expression described above, the positioning of reporter gene assays at the terminus of signalling pathways coupled with the complex nature of interactions within and between cellular signalling pathways would require a number of such assays to be performed in parallel to achieve effective discrimination between possible modes of action of a lead compound during the profiling process. Additionally the requirement to produce, maintain and deploy the large number of stably engineered cell lines for such an approach presents significant logistical issues (Pagliaro & Praestegaard, 2001, J Biomol Screen 6(3), 133-136).

To overcome the limitations of using stably engineered cells and hence to enable a more diverse set of assays to be applied in compound profiling a number of techniques have been developed to engineer cells to transiently express assay components. US 6544790 describes methods for producing arrays of transfected cells expressing nucleic acids by depositing a plurality of nucleic acid containing mixtures onto a surface in discrete, defined locations, allowing the mixtures to dry on the surface, and plating mammalian cells onto the surface under appropriate conditions for entry of the nucleic acids into the cells. This method allows a number of different assay reporters to be expressed in mammalian cells in parallel in what are commonly termed 'cell arrays'. Subsequent exposure of the cell array to a test compound can then be used to read out compound activity against a panel of cellular assays. While providing a high-throughput miniaturised format for lead profiling this method of producing cell arrays suffers from two drawbacks; the plasmid based chemical transfection method used is variable in transfection efficiency, restricting its use to a limited number of cell types and the fixed format of the printed array required limits the flexibility of the method to encompass changes in procedures and/or assays.

Adenoviral vectors are well known to have advantages over plasmid vectors, producing a higher percentage of cells expressing transgenic DNA and allowing a broader range of host cells to be transduced (Dietz et al., 1998, Blood, 91 (2), 392-8 ; Hatanaka et al., 2003, Mol Ther. 8(1):158-66.), and hence offer a more tractable route to deploying a wide range of lead profiling assays in mammalian cells. Adenoviral vectors have been extensively used in functional genomics to express proteins as a route to determining gene function. US 6413776 describes high-throughput methods for identifying the function of nucleic acids and their products by construction of a library of nucleic acids in adenoviral vectors, expression in a host cell to alter at least one phenotype and assigning a biological function to the product encoded by the nucleic acid correlated to the modified phenotype.

Some limited use of adenovirus vectors to transduce cells with reporter genes has also been described previously (Hartig et al., 2002, Toxicol Sci. 66(1):82-90). The authors developed an *in vitro* assay based upon a luciferase reporter system which requires cell lysis to evaluate a series of known endocrine-disrupting chemicals. Unfortunately, such *in vitro* techniques can be prone to artefacts resulting from cellular lysis and cannot be employed for real-time studies on living cells.

WO 01/81608 describes methods for transducing cells with a viral vector encoding a reporter gene the activity of which is increased in the presence of HIV proteins expressed from an HIV virus infecting the cell. An adenoviral vector comprising a GFP reporter under the control of an HIV inducible promoter is described, which may be used to detect the presence of HIV in a clinical sample or to screen for anti-HIV drugs. The method provides a means of detecting the presence of HIV infection and/or of screening for the effect of drugs on HIV by measuring the quantity of a single analyte.

None of the preceding prior art documents describes the parallel application of a diverse range of adenovirus encoded cellular assays targeted to a broad range of cellular pathways and processes to enable detailed profiling of compound function and activity in drug discovery.

### Summary of the Invention

According to a first aspect of the present invention, there is provided an *in vitro* method for characterising the activity and/or the function of a test agent on signalling pathways and/or cellular processes within a host cell, said method comprising the steps of
a) transiently expressing a plurality of virally encoded assays comprising a detectable protein from a first set of the assays in host cells located within a second set comprising a plurality of containers
b) optionally adding the test agent to one or more of the containers
c) performing one or more assay measurements
d) combining the assay measurements to produce a data set
e) analysing the data set to determine the functional characte istics of the test agent
characterised in that the assay measurements are conducted on living cells in a non-destructive manner.

The use of the word 'transiently' denotes that expression of the material introduced into the host cells by viral transduction is not stable in nature.

By 'plurality' is meant more than 1. Suitably, plurality means 2, 3, 4, 5, 6, 7, 8, 9 or 10. Preferably, plurality means 15, 20, 25, 30, 35, 40, 45 or 50. More preferably, plurality means 75, 100, 125, 150, 175 or 200.

The word 'set' is used to define a collection. The set may take the form of an array having a specific format (e.g. 96 well microplate) in which the location of each assay and/or container is known. In another embodiment the set may be a collection of assays or containers, each assay or container having a known location within the collection.

Suitably, one or more different virally encoded assays are expressed in the host cells in each of the containers.

Suitably, each container comprises different host cells. Optionally, each container comprises the same host cell or genetic variants of the same host cell.

Preferably, an adenoviral vector or a lentiviral vector is used to express the virally encoded assay in the host cells.

Suitably, a recombinant insect viral vector, for example BacMam (Ames R. et al. Receptors Channels. 2004;10(3-4):117-24.), is used to express the virally encoded assay in the host cells.

Suitably, the detectable protein is a fluorescent protein or a modified fluorescent protein. Fluorescent proteins and fluorescent protein derivatives of chromoproteins have been isolated from a wide variety of organisms, including *Aequoria victoria, Anemonia* species such as *A. majano* and *A. sulcata, Renilla* species, *Ptilosarcus* species, *Discosoma* species *Claularia* species, *Dendronephthyla species, Ricordia* species, *Scolymia* species, *Zoanthus* species, *Montastraea* species, *Heteractis* species, *Conylactis* species and *Goniopara* species.

Preferably, the fluorescent protein is a Green Fluorescent Protein (GFP) or a modified GFP. More preferably, the modified Green Fluorescent Protein (GFP) comprises one or more mutations selected from the group consisting of F64L. Y66H, Y66W, Y66F, S65T, S65A, V68L, Q69K, Q69M, S72A, T203I, E222G, V163A, I167T, S175G, F99S, M153T, V163A, F64L, Y145F, N149K, T203Y, T203Y, T203H, S202F and L236R.

Suitably, the detectable protein is an enzyme. Preferably, the enzyme is selected from the group consisting of β-galactosidase, nitroreductase, alkaline phosphatase and β-lactamase. The protein can thus be detected by the action of the enzyme on a suitable substrate added to the cell. Examples of such substrates include nitro-quenched CyDyes^{™} (GE Healthcare Bio-Sciences, nitroreductase substrate), ELF 97 (Molecular Probes, alkaline phosphate substrate) and CCF2 (Aurora Biosciences, β-lactamase substrate).

Suitably, the assay is selected from the group consisting of MAPKAP kinase 2, Glucocorticoid Receptor (GCCR), Epidermal Growth Factor (EGF), ERK1, Androgen Receptor (AR), STAT3, NFAT1, SMAD2, AKT1-PH, FYVE-PH, NFkB, PLC-PH and Rac1. MAPKAP kinase 2, ERK1, STAT3, NFAT1, SMAD2, AKT1-PH, FYVE-PH, PLC-PH and Rac1 Green Fluorescent Protein assays are commercially available (GE Healthcare Bio-Sciences, Little Chalfont, UK).

Suitably, the host cell is an eukaryotic cell, which cell may or may not be genetically modified. Preferably, the eukaryotic cell is a mammalian cell. More preferably, the host cell is a human cell.

Suitably, the test agent is a chemical agent or a physical agent.

Suitably, the chemical agent is an organic or inorganic compound. Such organic and inorganic compounds are tested in the method according to the invention as potential drugs or medicaments. The compounds are typically low molecular weight (less than 300 daltons) synthetic molecules. Preferably, the organic compound is selected from the group consisting of peptide, polypeptide, protein, carbohydrate, lipid, nucleic acid, polynucleotide and protein nucleic acid.

Suitably, the physical agent is electromagnetic radiation.

Suitably, step b) is omitted and the method is carried out in the absence of the test agent.

Suitably, the analysis step e) of the method of the invention comprises comparing each assay measurement determined in the presence of the test agent with the same assay measurement made in the absence of the test agent.

Optionally, the assay measurement in the absence of the test agent is known and is stored on a database. This reduces the time required to conduct the method of the invention and can thus increase screening throughput.

Preferably, the assay of the claimed method is measured using an imaging system, particularly where the assay is based upon a fluorescent reporter such as a Green Fluorscent Protein. Suitable imaging systems include the IN Cell Analyzer 3000 or the IN Cell Analyzer 1000 (GE Healthcare Bio-Sciences, Little Chalfont, UK).

According to a second aspect of the present invention, there is provided an automated system for characterising the activity and/or the function of a test agent on a population of cells comprising use of the method as hereinbefore described with an imaging system and a computerised data processing device.

In a third aspect of the present invention, there is provided a kit of parts comprising a plurality of virally encoded assays comprising a detectable protein for characterising the activity and/or function of a test agent on signalling pathways and/or cellular processes within a host cell.

Suitably, the virally encoded assays comprise adenoviral vectors or recombinant human insect viral vectors.

Preferably, the assay is selected from the group consisting of MAPKAP kinase 2, Glucocorticoid Receptor (GCCR), Epidermal Growth Factor (EGF), ERK1, Androgen Receptor (AR), STAT3, NFAT1, SMAD2, AKT1-PH, FYVE-PH, PLC-PH and Rac1.

Suitably, the detectable protein is a fluorescent protein or a modified fluorescent protein. Preferably, the fluorescent protein is a Green Fluorescent Protein (GFP) or a modified GFP. More preferably, the modified Green Fluorescent Protein (GFP) comprises one or more mutations selected from the group consisting of F64L, Y66H, Y66W, Y66F, S65T, S65A, V68L, Q69K, Q69M, S72A, T203I, E222G, V163A, I167T, S175G, F99S, M153T, V163A, F64L, Y145F, N149K, T203Y, T203Y, T203H, S202F and L236R.

Suitably, the detectable protein is an enzyme. Preferably, the enzyme is selected from the group consisting of β-galactosidase, nitroreductase, alkaline phosphatase and β-lactamase.

Preferably, the kit additionally comprises a substrate for the enzyme. The protein can thus be detected by the action of the enzyme on a suitable substrate added to the cell. Examples of such substrates include nitro-quenched CyDyes^{™} (GE Healthcare Bio-Sciences, nitroreductase substrate), ELF 97 (Molecular Probes, alkaline phosphate substrate) and CCF2 (Aurora Biosciences, β-lactamase substrate).

Most preferably, the enzyme is nitroreductase and the substrate is a substrate thereof.

### Brief Description of the Invention

In description of the method of the present invention reference is made to the following figures;
Figure 1: Preparation of cell arrays expressing virally encoded assays
Figure 2: Interrogation of a cellular signalling pathway using a set or an array of virally encoded assays
Figure 3: The EGFR-ERK cellular signalling pathway
Figure 4: Interrogation of multiple cellular signalling pathways using a set or an array of virally encoded assays
Figure 5: Data from a virally encoded MAPKAP kinase 2 signalling assay
Figure 6: Data from a virally encoded glucorticoid receptor signalling assay

### Detailed Description of the Invention

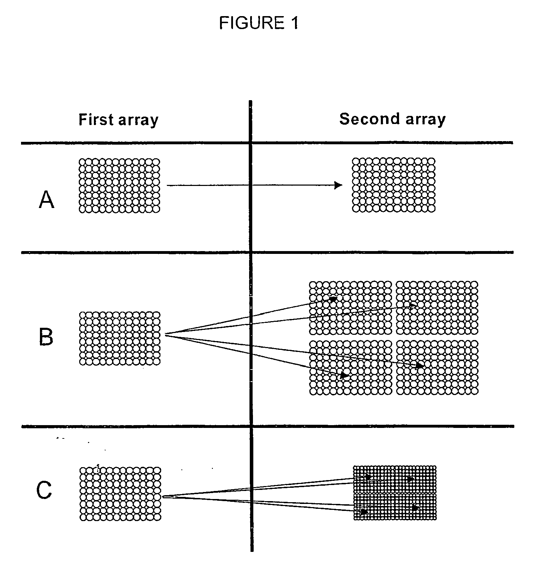

The method of the present invention may be deployed in a number of variations, some of which are shown in Figure 1:

Fig. 1A shows an application of the method of the invention wherein a first set or an array of encoded assays is used to transduce a second set or an array of cells where the first and second sets or arrays are identical in size and number of elements, for example replica plating of a 96 well first array to a 96 well second array containing a single cell type. Fig. 1B shows an application of the method of the invention wherein a first set or array of virally encoded assays is used to transduce a second set or array of cells where the second set or array is a 4-fold multiple of the first array while retaining the element size and spacing of the first array, for example replica plating from a 96 well first array to four identical 96 well second arrays, each second array containing a different cell type. Fig. 1C illustrates a third possible configuration wherein a first set or array of virally encoded assays is used to transduce a second set or array of cells where the second set or array is a four-fold multiple of the first array at a different element size and spacing from the first array, for example replica plating from a 96 well first array four times into a 384 well plate to provide four-fold replication for each assay.

These examples of varying embodiments of the invention illustrate significant flexibility in the application of the method to allow different approaches to profiling of lead compounds. Further flexibility in the method is illustrated by the examples given in Table 1.

**Table 1**

| First array | Second array | |
|---|---|---|
| Assays | Host cell types | Test compounds |
| N | 1 | 1 |
| N | 1 | N |
| N | n | 1 |
| N | n | N |

In the method of the invention the first set or array comprises virally encoded assays disposed in a set or an array of discrete containers, typically a 96 well microplate. The number of different analytes represented by these assays can be between 2 and N, where N is the number of elements in the first array (i.e. N = 2 to 96 for a 96 well plate). In the method of the invention the virally encoded assays are applied to cells growing in a second array of discrete containers, for example a second 96 well plate. The number of cell types growing in the second array can be between 1 and n, where n is the number of elements in the second array (i.e. n =1 to 96 for a 96 well plate). These variations in first and second array formats can be combined with variable numbers of test compounds where the number of test compounds applied to the second array varies inversely with the number of assays of the first array. For example, for a case where the first array comprises a 96 well plate of 96 separate assays which is used to prepare a second array using a single cell type, the second array may be used to conduct a 96 assay profile of a single test compound (N:1:1 in table 1).

Other variations are also possible, for example where different cell host types are required for assays due to differing expression of endogenous pathway components between cell types, in these cases a first set or array comprising 96 separate assays may be used to prepare a second set or array using several cell types (from 2 to 96 in this example) which is subsequently used to profile a single test compound (N:n:1 in table 1).

If the aim of the profiling study is to determine the effect of genetic background on test compound activity or function, a further variation of the method can be applied in which, for example, the first array comprises a 96 well plate containing two different assays and this array is used to prepare a second array using 48 different cell types, the second array may be used to profile a single test compound in 48 genetic backgrounds (N:n:1 in table 1). The above examples are not intended to be restrictive, and further possible variations will be apparent to those skilled in the art.

Recombinant adenoviral vectors for use in the method of the present invention may be generated by several methods (Wang & Huang, 2000, Drug Discovery, Today 5, 10-16.). One of the most common methods, and the method used in the examples cited below, is based on the two plasmid rescue method. This method has been described in detail elsewhere (Bett et al., 1993, PNAS 91, 8802-8806; US 6140087 Adenovirus vectors for gene therapy).

cDNAs (e.g. MAPKAPkinase2, or the human glucocorticoid receptor - GCCR, fused to the cDNA encoding GFP) were cloned into the expression cassette of the first nucleic acid constructs, called 'shuttle plasmids'. Shuttle plasmids contain the left approximately 340 nucleotides of the adenoviral type 5 genome including the packaging signal and inverted terminal repeat (ITR) sequences, a polycloning site and viral sequences from near the right end of the adenoviral E1 gene region. The second nucleic acid constructs are relatively large and are known as pBHG10 (Bett et al., 1993, PNAS 91, 8802-8806).

The pBHG10 plasmid contains most of the viral genome and would be capable of producing infectious virus but for the deletion of the packaging signal and parts of the E1 gene region. The viral sequences from near the right end of E1 in the shuttle plasmid overlap with sequences in the pBHG10 plasmid. Cloning was carried out using standard laboratory techniques. Both plasmids were expanded by transforming bacteria and then growing overnight in LB. The bacteria were precipitated by centrifugation and processed by a modified alkaline lysis procedure (Sambrook et al. ,1989, Molecular Cloning: A Laboratory Manual (Cold Spring Harbour Lab. Press) 2nd edition). The first and second nucleic acid constructs were co-transfected into HEK 293 cells (adenovirus type 5 transformed human embryonic kidney cell line) by standard calcium phosphate co-precipitation ( Sambrook et al. ,1989, Molecular Cloning: A Laboratory Manual (Cold Spring Harbour Lab. Press) 2nd edition). One day after transfections the cells were overlaid with tissue culture medium (DMEM supplemented with 10% FCS) containing 0.5% agarose. Co-transfection of the two plasmids into HEK 293 cells lead to homologous recombination between the overlapping sequences in the first and second nucleic acid constructs. The resulting recombinant adenoviral vectors contain the packaging signal derived from the shuttle plasmids as well as the cDNA cloned into the shuttle plasmid polycloning site. Neither plasmid has the capacity to produce replicating virus and viral progeny can only be produced as a result of recombination. The deletions in the E1 region are complemented by constitutive production in the 293 cells. Recombinant adenoviral DNA is able to replicate and propagate in E1 complementing packaging cells to produce recombinant adenoviral vector. After approximately 2 weeks recombinant adenoviral vectors formed plaques in the 293 cells. These were observed macroscopically under the agarose containing culture medium. To generate adenoviral vectors of adequate titre for the experiments described below, stocks were generated by expanding the isolated plaques. The plaques were removed from the cultures with a sterile pipette tip and added to 200ul of PBS and added to near confluent monolayers of 293 cells (E1 complementing packaging cells). After approximately 48 hours the cells became detached as a result of infection with the recombinant adenovirus vector and were harvested into the tissue culture medium. The adenovirus vector was released from the producer cells by three cycles of flash freeze/thawing. The lysates were cleared by centrifugation at 3000rpm for 10 minutes and the recombinant adenoviral vectors purified by anion exchange chromatography
(www.bdbiosciences.com/clontech/products/cat/HTML/1260.shtml).
Assessment of the concentration of recombinant adenoviral vectors was made using an immunocytochemical test (www.bdbiosciences.com/clontech/archive/ APR02UPD/pdf/Adeno-X.pdf). The recombinant adenoviral vector can be introduced into target cells which are grown to allow sufficient expression of the product encoded by the cDNA cloned into the vector. Expression of the protein fused to GFP allows detection of and analysis of a biological activity of the protein. In the case of the two proteins described in the examples below (MAPKAP kinase 2 and GCCR), biological activity can be assessed by translocation of the GFP fused to the gene from one region of the cell to another (Roquemore et al., 2002, Life Science News 11, 1-5 Amersham Biosciences). Identification of a biological effect by a compound in a library can result in a small molecule drug for the treatment of human disease.

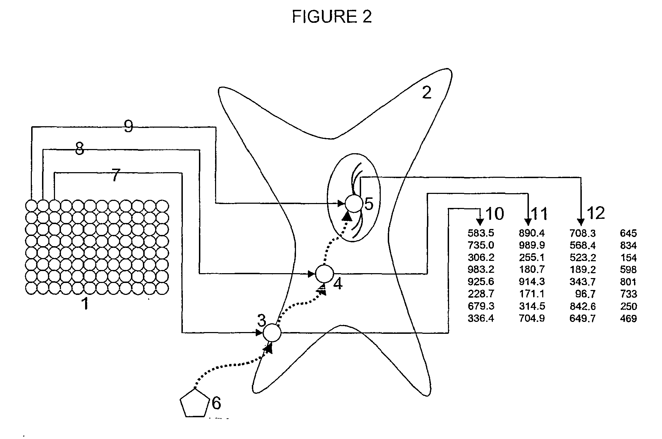

The application of one embodiment of the present invention using a set or an array of separate virally encoded assays to profiling of lead compound activity in cellular assays is illustrated by the representative example shown in Fig. 2. An array of virally encoded assays [1] is used to express a range of sensors of cellular pathways and processes in host cells [2]. A first sensor [7] monitors activation of a cell surface receptor protein [3] which activates a second protein [4] monitored by a second sensor [8], which in turn activates gene expression [5] monitored by a third sensor [9]. This combination of virally encoded sensors allows activity of a test compound [6] against the cellular pathway comprising components [3], [4] and [5] to be determined by analysis of the readouts from the three sensors [10], [11] and [12]. In this embodiment of the present invention further virally encoded assays provided in the array [1] are used to address further additional cellular pathways and processes in parallel.

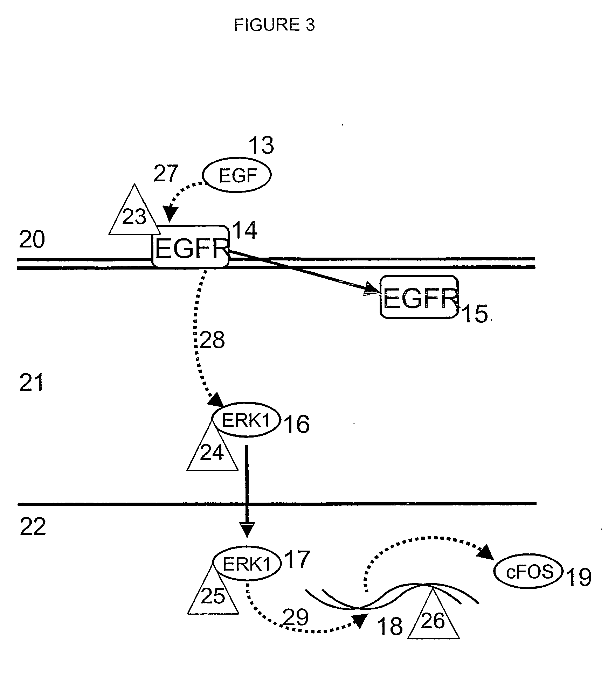

This embodiment of the current invention represents a generic approach which can be applied to determining the activity of a test compound against a range of cellular pathways. Many cellular signalling pathways exist to transduce a signal arriving at a cell surface receptor via intermediate molecules to a final gene expression event (Gomperts et al., Signal Transduction, Academic Press 2003). A typical example of such a pathway originating from the Epidermal Growth Factor (EGF) receptor with sensing points applied using virally encoded assays is shown in Fig. 3. The EGF receptor (EGFR) [14] resides in the cytoplasmic membrane [20] of EGF responsive cells where it available to bind EGF. Binding of EGF [13] causes receptor activation, transduction of signal to ERK1 [16] in the cell cytoplasm [21] and internalisation of the EGFR [15]. Once activated ERK1 translocates from the cytoplasm to the nucleus [22] where the activated ERK1 [17] promotes gene expression [18] from ERK1 responsive control elements leading to the expression of cFOS [19] and other genes. This signalling pathway therefore has three events for which virally encoded assays can be engineered to report signal transduction along the signalling pathway; receptor internalisation [23], ERK1 translocation [24] and ERK1 induced gene expression [26].

EGFR internalisation may be measured by viral expression of an engineered receptor tagged with a pH responsive fluorescent molecule which changes fluorescence when the receptor is internalised into acidic vesicles (Adie et al., 2002, Biotechniques 33(5):1152-4, 1156-7.). ERK1 translocation may be measured by viral expression of a GFP-ERK1 fusion protein (Chamberlain & Hahn, 2000 Traffic Oct; 1(10):755-62.). ERK1 induced gene expression may be measured by viral expression of a reporter gene under the control of an ERK1 responsive promoter. WO0157237 describes suitable methods for monitoring reporter gene expression in living cells. Such assays can be measured quantitatively by sub-cellular imaging and image analysis. US 6400487 provides methods and apparatus for screening large numbers of chemical compounds and performing a wide variety of fluorescent assays, including live cell assays. The methods utilise a laser linescan confocal microscope with high speed, high resolution and multi-wavelength capabilities and real time data-processing that are suitable for measurement of the assays of the present invention.

In the method of the present invention the signalling pathway of Fig.3 would be probed by three separate virally encoded assays each expressed in a separate cell population in a separate well of a microwell plate to provide a means for profiling the activity of a test compound. Three possible points of activity of a test compound are shown on Fig. 3; at the level of the EGF receptor [27], at the level of ERK1 [28] and at the level of gene expression [29]. Consequently, by combination and examination of data from individual assays it is possible to determine the point of action of test compounds active against the pathway. For example, a test compound showing positive results for GFP translocation [24] and gene expression [25] but not receptor activation [23] is acting downstream of the receptor. Distinguishing stimulatory or inhibitory activity of the test compound is readily achieved by performing replicate assays in the presence and absence of EGF. For example, a test compound which when tested in the presence of EGF shows negative results for gene expression [25] but shows positive results in assays [23] and [24] is inhibiting the pathway at the level of gene expression.

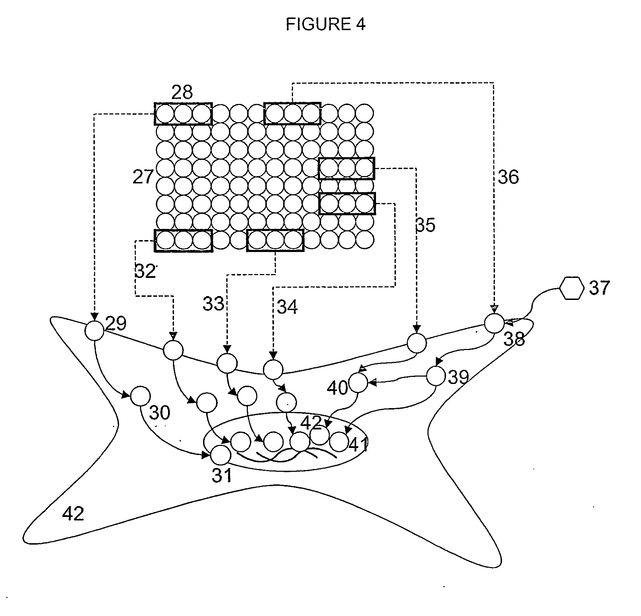

The method of the present invention allows many such signalling pathways to be interrogated simultaneously in parallel by expression of sets or arrays of virally encoded assays for specific points in separate and overlapping signalling pathways. The data from these arrays of assays allow the activity and specificity of a compound to be profiled across a wide range of biological pathways, this process is outlined schematically in Fig. 4.

To achieve profiling of a compound a set or an array of virally encoded assays [27] is expressed in a corresponding set or array of cell populations such that each component of the assay set or array is expressed in a separate cell population.
For each cell population each cell [42] will report on one element of a specific cellular pathway or process. Within the assay array and the corresponding cell array, sub-elements of the array [28] will address individual components of a defined pathway or process wherein each assay within the sub-element [28] addresses a separate but linked component [29], [30] and [31] to report signal transduction as described above for Fig. 3. Further sub-elements within the arrays address further pathways and processes [32], [33], [34], [35] and [36]. Exposure of multiple populations of cells within the array, each cell expressing a separate virally encoded assay, to a test compound [37] allows the activity and specificity of the test compound to be determined by examination of the data produced by all assays in the array. A test compound [37] acting at a cell surface receptor [38] coupled to a signal transduction molecule [39] which is in turn coupled to activation of gene expression [41] and also to a second transduction molecule [40] which is also coupled to gene expression [42] will give rise to positive assay signals in cells in which sensors are expressed which report activity at points [38], [39], [40], [41], and [42] by means as described above for Fig. 3. In the same array absence of signals from array sub-elements [28], [32], [33] and [34] addressing other signalling pathways and components indicates inactivity of the test compound towards these pathways and confirms specificity to the interacting pathways addressed by sub-elements [35] and [36].

### Specific Examples

The following examples describe assay procedures using recombinant adenoviral vectors for single 96 well plates of target cells.

48 hours before the assay, logarithmically growing HeLa cells were detached by treatment with trypsin. The cells were counted and adjusted to 1 X 10⁶/ml, and adjusted with culture medium to a final volume of 20ml. Recombinant adenoviral vector particles (see Examples 1 & 2 below) were added to the cells at a rate of 100 per cell (this number is referred to as multiplicity of infection (MOI)). The cells and the virus were mixed and plated into a tissue culture flask. 24 hours iater the cells were detached from the cuture plate (as described above). The cells were seeded at 4 x 10³ cells/100 µl into wells of a 96 well plate. After a further 24 hours in culture the cells were assayed as described below.

### Example 1 : MAPKAP kinase 2 Assay

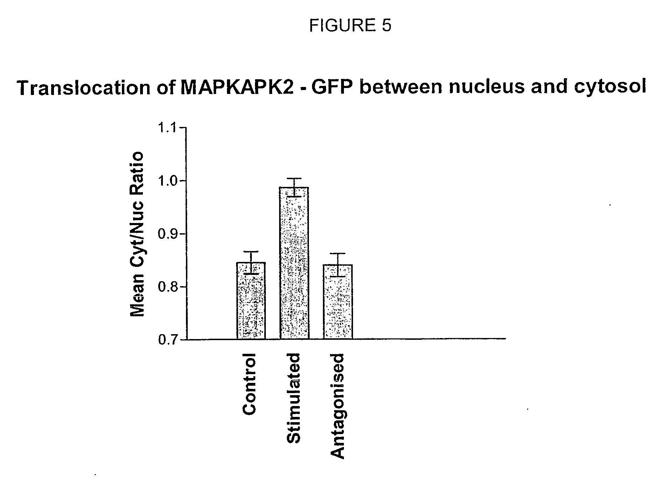

The assay described includes both antagonism and agonism of the process in which MAPKAP kinase 2 translocates from the nucleus to the cytoplasm. Compounds of a library may replace either the agonist or the antagonist when attempting to identify compounds for the treatment of human disease. The culture medium was decanted from the cells. In some wells, the culture medium was replaced with fresh culture medium. In other wells the medium was replaced with medium containing 300 nanomolar anisomycin (agonist). In other wells the medium was replaced with medium containing agonist and antagonist (100 micromolar SB203580-HCl). The cells were incubated for 90 minutes at 37°C. The medium was decanted from the cells and they were fixed with 2% formalin solution for 15 minutes. The cells were then washed with PBS and the nuclei stained by the addition of a 2.5 micromolar solution of Hoechst 33342. The results of the assay are shown in Figure 5.

### Example 2: Glucocorticoid Receptor Assay

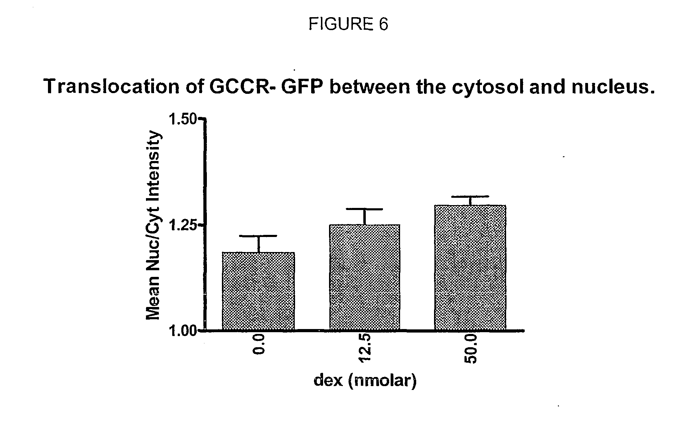

The second assay illustrates agonism of the process by which the glucocorticoid receptor (GR) is translocated from the cytoplasm to the nucleus. HeLa cells were set up as described above with 100 MOI of the recombinant adenoviral vector containing the GR linked to GFP. 1 hour prior to the assay, tissue culture medium was replaced with medium containing charcoal stripped serum for 1 hour at 37°C. This medium was decanted and replaced with medium containing the same serum and a variable concentration of dexamethasone (800, 400, 200, 100, 50, 25nmolar), for 20 minutes at 37°C. Compounds from a library that may affect the translocation of GR from the cytoplasm to the nucleus may be included. The medium was then decanted from the cells and they were fixed and processed as described above. The results of the assay are shown in Figure 6.

## Claims

1. An *in vitro* method for characterising the activity and/or the function of a test agent on signalling pathways and/or cellular processes within a host cell, said method comprising
a) transiently expressing a plurality of virally encoded assays comprising a detectable protein from a first set of said assays in host cells located within a second set comprising a plurality of containers
b) optionally adding said test agent to one or more of said containers
c) performing one or more assay measurements
d) combining said assay measurements to produce a data set
e) analysing said data set to determine the functional characteristics of the test agent
**characterised in that** said assay measurements are conducted on living cells in a non-destructive manner.

2. The method according to claim 1, wherein one or more different virally encoded assays are expressed in the host cells in each of the containers.

3. The method according to any preceding claim, wherein each container comprises different host cells.

4. The method according to any of claims 1 to 2, wherein each container comprises the same host cell or genetic variants of said same host cell.

5. The method according to any preceding claim, wherein an adenoviral vector is used to express the virally encoded assay in the host cells.

6. The method according to any of claims 1 to 4, wherein a recombinant human insect viral vector is used to express the virally encoded assay in the host cells.

7. The method according to any preceding claim, wherein said detectable protein is a fluorescent protein or a modified fluorescent protein.

8. The method according to claim 7, wherein said fluorescent protein is a Green Fluorescent Protein (GFP) or a modified GFP.

9. The method according to claim 8, wherein said modified Green Fluorescent Protein (GFP) comprises one or more mutations selected from the group consisting of F64L, Y66H, Y66W, Y66F, S65T, S65A, V68L, Q69K, Q69M, S72A, T2031, E222G, V163A, I167T, S175G, F99S, M153T, V163A, F64L, Y145F, N149K, T203Y, T203Y, T203H, S202F and L236R.

10. The method according to any preceding claim, wherein the detectable protein is an enzyme.

11. The method according to claim 10, wherein said enzyme is selected from the group consisting of β-galactosidase, nitroreductase, alkaline phosphatase and β-lactamase.

12. The method according to any preceding claim, wherein the assay is selected from the group consisting of MAPKAP kinase 2, Glucocorticoid Receptor (GCCR), Epidermal Growth Factor (EGF), ERK1, Androgen Receptor (AR), STAT3, NFAT1, SMAD2, AKT1-PH, FYVE-PH, PLC-PH and Rac1.

13. The method according to any of claims 1 to 12, wherein said host cell is an eukaryotic cell, which cell may or may not be genetically modified.

14. The method according to claim 13, wherein said eukaryotic cell is a mammalian cell.

15. The method according to claim 13 or 14, wherein the host cell is a human cell.

16. The method according to any preceding claim, wherein the test agent is a chemical agent or a physical agent.

17. The method according to claim 16, wherein said chemical agent is an organic or inorganic compound.

18. The method according to claim 17, wherein said organic compound is selected from the group consisting of peptide, polypeptide, protein, carbohydrate, lipid, nucleic acid, polynucleotide and protein nucleic acid.

19. The method according to claim 16, wherein said physical agent is electromagnetic radiation.

20. The method according to any preceding claim, wherein step b) is omitted and the method is carried out in the absence of the test agent.

21. The method according to any of claims 1 to 19, wherein the analysis step e) comprises comparing each assay measurement determined in the presence of the test agent with the same assay measurement made in the absence of the test agent.

22. The method according to claim 21, wherein the assay measurement in the absence of the test agent is known and is stored on a database.

23. The method according to any preceding claim, wherein the assay is measured using an imaging system.

24. The method according to claim 23, wherein said imaging system is the IN Cell Analyzer 3000 or the IN Cell Analyzer 1000.

25. An automated system for characterising the activity and/or the function of a test agent on a population of cells comprising use of the method according to any of claims 1 to 24 together with an imaging system and a computerised data processing device.

26. A kit of parts comprising a plurality of virally encoded assays comprising a detectable protein for characterising the activity and/or function of a test agent on signalling pathways and/or cellular processes within a host cell.

27. The kit of parts according to claim 26, wherein said virally encoded assays comprise adenoviral vectors or recombinant human insect viral vectors.

28. The kit of parts according to either of claims 26 or 27, wherein the assay is selected from the group consisting of MAPKAP kinase 2, Glucocorticoid Receptor (GCCR), Epidermal Growth Factor (EGF), ERK1, Androgen Receptor (AR), STAT3, NFAT1, SMAD2, AKT1-PH, FYVE-PH, PLC-PH and Rac1.

29. The kit of parts according to any of claims 26 to 28, wherein said detectable protein is a fluorescent protein or a modified fluorescent protein.

30. The kit of parts according to any of claims 26 to 28, wherein the detectable protein is an enzyme.

31. The kit of parts according to claim 30, wherein said enzyme is selected from the group consisting of β-galactosidase, nitroreductase, alkaline phosphatase and β-lactamase.

32. The kit of parts according to either of claims 30 or 31, wherein the kit additionally comprises a substrate for the enzyme.

33. The kit of parts according to claim 32, wherein the enzyme is nitroreductase and said substrate is a substrate thereof.

## Patentansprüche

1. In vitro-Verfahren zum Charakterisieren der Aktivität und/oder der Funktion eines Testmittels auf Signalgebungswege und/oder zelluläre Prozesse innerhalb einer Wirtszelle, wobei das Verfahren umfasst
a) transientes Exprimieren einer Mehrzahl von viralkodierten Assays, umfassend ein detektierbares Protein aus einem ersten Satz der Assays in Wirtszellen, lokalisiert innerhalb eines zweiten Satzes, der eine Mehrzahl von Behältern umfasst
b) gegebenenfalls Zugeben des Testmittels zu einem oder mehreren der Behälter
c) Ausführen von einer oder mehreren Assay-Messungen
d) Kombinieren der Assay-Messungen, um einen Datensatz herzustellen
e) Analysieren des Datensatzes, um die funktionellen Eigenschaften des Testmittels zu bestimmen
**dadurch gekennzeichnet, dass** die Assay-Messungen ausgeführt werden mit lebenden Zellen auf eine nicht-zerstörende Weise.

2. Verfahren nach Anspruch 1, wobei einer oder mehrere unterschiedlich viralkodierte Assays in den Wirtszellen in jedem der Behälter exprimiert werden.

3. Verfahren nach einem der vorstehenden Ansprüche, wobei jeder Behälter verschiedene Wirtszellen umfasst.

4. Verfahren nach einem der Ansprüche 1 bis 2, wobei jeder Behälter dieselbe Wirtszelle oder genetische Varianten derselben Wirtszelle umfasst.

5. Verfahren nach einem der vorstehenden Ansprüche, wobei ein adenoviraler Vektor verwendet wird, um den viralkodierten Assay in den Wirtszellen zu exprimieren.

6. Verfahren nach einem der Ansprüche 1 bis 4, wobei ein rekombinanter menschlicher Insekten-Virus-Vektor verwendet wird, um den viralkodierten Assay in den Wirtszellen zu exprimieren.

7. Verfahren nach einem der vorstehenden Ansprüche, wobei das detektierbare Protein ein fluoreszierendes Protein oder ein modifiziertes fluoreszierendes Protein ist.

8. Verfahren nach Anspruch 7, wobei das fluoreszierende Protein ein Grün-Fluoreszierendes-Protein (GFP) oder ein modifiziertes GFP ist.

9. Verfahren nach Anspruch 8, wobei das modifizierte Grün-Fluoreszierende-Protein (GFP) eine oder mehrere Mutationen umfasst, ausgewählt aus der Gruppe, die besteht aus F64L, Y66H, Y66W, Y66F, S65T, S65A, V68L, Q69K, Q69M, S72A, T203I, E222G, V163A, I167T, S175G, F99S, M153T, V163A, F64L, Y145F, N149K, T203Y, T203Y, T203H, S202F und L236R.

10. Verfahren nach einem der vorstehenden Ansprüche, wobei das detektierbare Protein ein Enzym ist.

11. Verfahren nach Anspruch 10, wobei das Enzym ausgewählt ist aus der Gruppe, die besteht aus β-Galaktosidase, Nitroreduktase, alkalischer Phosphatase und β-Lactamase.

12. Verfahren nach einem der vorstehenden Ansprüche, wobei der Assay ausgewählt ist aus der Gruppe, die besteht aus MAPKAP-Kinase 2, Glucocorticoid-Rezeptor (GCCR), epidermaler Wachstumsfaktor (EGF), ERK1, Androgen-Rezeptor (AR), STAT3, NFAT1, SMAD2, AKT1-PH, FYVE-PH, PLC-PH und Rac1.

13. Verfahren nach einem der Ansprüche 1 bis 12, wobei die Wirtszelle eine eukaryontische Zelle ist, wobei die Zelle genetisch modifiziert sein kann oder nicht.

14. Verfahren nach Anspruch 13, wobei die eukaryontische Zelle eine Säugerzelle ist.

15. Verfahren nach Anspruch 13 oder 14, wobei die Wirtszelle eine menschliche Zelle ist.

16. Verfahren nach einem der vorstehenden Ansprüche, wobei das Testmittel ein chemisches Mittel oder ein physikalisches Mittel ist.

17. Verfahren nach Anspruch 16, wobei das chemische Mittel eine organische oder anorganische Verbindung ist.

18. Verfahren nach Anspruch 17, wobei die organische Verbindung ausgewählt ist aus der Gruppe, die besteht aus Peptid, Polypeptid, Protein, Kohlenhydrat, Lipid, Nukleinsäure, Polynukleotid und Proteinnukleinsäure.

19. Verfahren nach Anspruch 16, wobei das physikalische Mittel eine elektromagnetische Strahlung ist.

20. Verfahren nach einem der vorstehenden Ansprüche, wobei Schritt b) weggelassen wird und das Verfahren ausgeführt wird in Abwesenheit des Testmittels.

21. Verfahren nach einem der Ansprüche 1 bis 19, wobei der Analyseschritt e) umfasst Vergleichen jeder Assay-Messung, die in Anwesenheit des Testmittels bestimmt wird, mit derselben Assay-Messung, die in Abwesenheit des Testmittels ausgeführt wird.

22. Verfahren nach Anspruch 21, wobei die Assay-Messung in Abwesenheit des Testmittels bekannt und gespeichert ist in einer Datenbank.

23. Verfahren nach einem der vorstehenden Ansprüche, wobei der Assay ausgeführt wird unter Verwenden eines Bildgebungssystems.

24. Verfahren nach Anspruch 23, wobei das Bildgebungssystem der IN-Cell-Analyzer 3000 oder der IN-Cell-Analyzer 1000 ist.

25. Automatisiertes System zum Charakterisieren der Aktivität und/oder Funktion eines Testmittels auf einer Population von Zellen, umfassend die Anwendung des Verfahrens nach einem der Ansprüche 1 bis 24 zusammen mit einem Bildgebungssystem und einer Computer-implementierten Datenverarbeitungsvorrichtung.

26. Kit aus Teilen, umfassend eine Vielzahl von viralkodierten Assays, umfassend ein detektierbares Protein zum Charakterisieren der Aktivität und/oder Funktion eines Testmittels auf Signalgebungswege und/oder zelluläre Prozesse innerhalb einer Wirtszelle.

27. Kit ausTeilen nach Anspruch 26, wobei die viralkodierten Assays adenovirale Vektoren oder rekombinante menschliche Insekten-Virus-Vektoren umfassen.

28. Kit aus Teilen nach einem der Ansprüche 26 oder 27, wobei der Assay ausgewählt ist aus der Gruppe, die besteht aus MAPKAP-Kinase 2, Glucocorticoid-Rezeptor (GCCR), epidermaler Wachstumsfaktor (EGF), ERK1, Androgen-Rezeptor (AR), STAT3, NFAT1, SMAD2, AKT1-PH, FYVE-PH, PLC-PH und Rac1.

29. Kit aus Teilen nach einem der Ansprüche 26 bis 28, wobei das detektierbare Protein ein fluoreszierendes Protein oder ein modifiziertes fluoreszierendes Protein ist.

30. Kit von Teilen nach einem der Ansprüche 26 bis 28, wobei das detektierbare Protein ein Enzym ist.

31. Kit aus Teilen nach Anspruch 30, wobei das Enzym ausgewählt ist aus der Gruppe, die besteht aus β-Galactosidase, Nitroreduktase, alkalischer Phosphatase und β-Lactamase.

32. Kit aus Teilen nach einem der Ansprüche 30 oder 31, wobei das Kit zusätzlich ein Substrat für das Enzym umfasst.

33. Kit aus Teilen nach Anspruch 32, wobei das Enzym Nitroreduktase ist und das Substrat ein Substrat davon ist.

## Revendications

1. Procédé *in vitro* permettant de caractériser l'activité et/ou la fonction d'un agent test sur des voies de signalisation et/ou des processus cellulaires à l'intérieur d'une cellule hôte, ledit procédé comprenant
a) l'expression transitoire d'une pluralité de dosages à codage viral comprenant une protéine détectable à partir d'un premier ensemble desdits dosages dans des cellules hôtes situées à l'intérieur d'un second ensemble comprenant une pluralité de récipients,
b) l'ajout éventuel dudit agent test à un ou plusieurs desdits arécipients,
c) la réalisation d'une ou de plusieurs mesures par dosage,
d) la combinaison desdites mesures par dosage pour produire un ensemble de données,
e) l'analyse desdites données pour déterminer les caractéristiques fonctionnelles de l'agent test,
**caractérisé en ce que** lesdites mesures par dosage sont mises en oeuvre sur des cellules vivantes de manière non destructrice.

2. Procédé selon la revendication 1, dans lequel un ou plusieurs dosages à codage viral différents sont exprimés dans les cellules hôtes dans chacun des récipients.

3. Procédé selon l'une quelconque des revendications précédentes, dans lequel chaque récipient comprend différentes cellules hôtes.

4. Procédé selon l'une quelconque des revendications 1 à 2, dans lequel chaque récipient comprend la même cellule hôte ou des variants génétiques de ladite même cellule hôte.

5. Procédé selon l'une quelconque des revendications précédentes, dans lequel un vecteur d'adénovirus est utilisé pour exprimer le dosage à codage viral dans les cellules hôtes.

6. Procédé selon l'une quelconque des revendications 1 à 4, dans lequel un vecteur de virus d'insecte recombinant humain est utilisé pour exprimer le dosage à codage viral dans les cellules hôtes.

7. Procédé selon l'une quelconque des revendications précédentes, dans lequel ladite protéine détectable est une protéine fluorescente ou une protéine fluorescente modifiée.

8. Procédé selon la revendication 7, dans lequel ladite protéine fluorescente est une Protéine Fluorescente Verte (GFP) ou une GFP modifiée.

9. Procédé selon la revendication 8, dans lequel ladite Protéine Fluorescente Verte (GFP) modifiée comprend une ou plusieurs mutations choisies dans le groupe constitué par F64L, Y66H, Y66W, Y66F, S65T, S65A, V68L, Q69K, Q69M, S72A, T203I, E222G, V163A, I167T, S175G, F99S, M153T, V163A, F64L, Y145F, N149K, T203Y, T203Y, T203H, S202F et L236R.

10. Procédé selon l'une quelconque des revendications précédentes, dans lequel la protéine détectable est une enzyme.

11. Procédé selon la revendication 10, dans lequel ladite enzyme est choisie dans le groupe constitué par la β-galactosidase, la nitroréductase, la phosphatase alcaline et la β-lactamase.

12. Procédé selon l'une quelconque des revendications précédentes, dans lequel le dosage est choisi dans le groupe constitué par la MAPKAP kinase 2, le récepteur du glucocorticoïde (GCCR), le facteur de croissance épidermique (EGF), ERK1, le récepteur d'androgène (AR), STAT3, NFAT1, SMAD2, AKT1-PH, FYVE-PH, PLC-PH et Rac1.

13. Procédé selon l'une quelconque des revendications 1 à 12, dans lequel ladite cellule hôte est une cellule eucaryote, laquelle cellule peut être génétiquement modifiée ou non.

14. Procédé selon la revendication 13, dans lequel ladite cellule eucaryote est une cellule de mammifère.

15. Procédé selon la revendication 13 ou 14, dans lequel la cellule hôte est une cellule humaine.

16. Procédé selon l'une quelconque des revendications précédentes, dans lequel l'agent test est un agent chimique ou un agent physique.

17. Procédé selon la revendication 16, dans lequel ledit agent chimique est un composé organique ou inorganique.

18. Procédé selon la revendication 17, dans lequel ledit composé organique est choisi dans le groupe constitué par peptide, polypeptide, protéine, hydrate de carbone, lipide, acide nucléique, polynucléotide et acide nucléique protéique.

19. Procédé selon la revendication 16, dans lequel ledit agent physique est un rayonnement électromagnétique.

20. Procédé selon l'une quelconque des revendications précédentes, dans lequel l'étape b) est omise et le procédé est effectué en l'absence de l'agent test.

21. Procédé selon l'une quelconque des revendications 1 à 19, dans lequel l'étape d'analyse e) comprend la comparaison de chaque mesure par dosage déterminée en présence de l'agent test avec la même mesure par dosage réalisée en l'absence de l'agent test.

22. Procédé selon la revendication 21, dans lequel la mesure par dosage en l'absence de l'agent test est connue et est mise en mémoire dans une banque de données.

23. Procédé selon l'une quelconque des revendications précédentes, dans lequel le dosage est mesuré au moyen d'un système d'imagerie.

24. Procédé selon la revendication 23, dans lequel ledit système d'imagerie est l'analyseur cellulaire IN Cell Analyzer 3000 ou IN Cell Analyzer 1000.

25. Système automatisé permettant de caractériser l'activité et/ou la fonction d'un agent test sur une population de cellules comprenant l'utilisation du procédé selon l'une quelconque des revendications 1 à 24 conjointement à un système d'imagerie et à un dispositif de traitement de données assisté par ordinateur.

26. Kit d'éléments comprenant une pluralité de dosages à codage viral comprenant une protéine détectable permettant de caractériser l'activité et/ou la fonction d'un agent test sur des voies de signalisation et/ou des processus cellulaires à l'intérieur d'une cellule hôte.

27. Kit d'éléments selon la revendication 26, dans lequel lesdits dosages à codage viral comprennent des vecteurs d'adénovirus ou des vecteurs de virus d'insecte recombinants humains.

28. Kit d'éléments selon l'une ou l'autre des revendications 26 ou 27, dans lequel le dosage est choisi dans le groupe constitué par la MAPKAP kinase 2, le récepteur du glucocorticoïde (GCCR), le facteur de croissance épidermique (EGF), ERK1, le récepteur d'androgène (AR), STAT3, NFAT1, SMAD2, AKT1-PH, FYVE-PH, PLC-PH et Rac1.

29. Kit d'éléments selon l'une quelconque des revendications 26 à 28, dans lequel ladite protéine détectable est une protéine fluorescente ou une protéine fluorescente modifiée.

30. Kit d'éléments selon l'une quelconque des revendications 26 à 28, dans lequel la protéine détectable est une enzyme.

31. Kit d'éléments selon la revendication 30, dans lequel ladite enzyme est choisie dans le groupe constitué par la β-galactosidase, la nitroréductase, la phosphatase alcaline et la β-lactamase.

32. Kit d'éléments selon l'une ou l'autre des revendications 30 ou 31, dans lequel le kit comprend en outre un substrat destiné à l'enzyme.

33. Kit d'éléments selon la revendication 32, dans lequel l'enzyme est la nitroréductase et ledit substrat est un substrat de celle-ci.
